# EUROPEAN PATENT SPECIFICATION

(11) **EP 1 039 905 B1**
(45) Date of publication and mention of the grant of the patent: **05.07.2006**
(21) Application number: 98957330.8
(22) Date of filing: 14.09.1998
(51) Int. Cl.: A61K 31/4439, A61P 1/04, A61K 47/42

(54) **PHARMACEUTICAL FORMULATION COMPRISING GLYCINE AS A STABILIZER**
PHARMAZEUTISCHE ZUSAMMENSETZUNG ENTHALTEND GLYZIN ALS STABILISATOR
FORMULATION PHARMACEUTIQUE COMPRENANT DE LA GLYCINE COMME STABILISANT

(30) Priority: 14.10.1997 US 62089
(43) Date of publication of application: 04.10.2000
(73) Proprietor: Eisai Co., Ltd., Tokyo 112-88 (JP)
(72) Inventor: MCSHANE, James, Durham, NC 27713 (US); WOOD, Ray, Durham, NC 27713 (US); WATANABE, Sumio Eisai Co., Ltd., Hashima Gifu 501-61 (JP); IWAMOTO, Kiyoshi, Gifu (JP); ONAI, Katsumi Famile Moridou, Kitakata, Ichinomiya Aichi (JP)
(74) Representative: Howard, Paul Nicholas
(86) International application number: PCT/US1998/021972
(87) International publication number: WO 1999/018959

(56) References cited:
- EP-A- 0 082 481
- EP-A- 0 268 956
- WO-A-95/28951
- DE-A- 3 000 743
- DATABASE WPI Section Ch, Week 9335 Derwent Publications Ltd., London, GB; Class B02, AN 93-278196 XP002090491 & JP 05 194225 A (YOSHITOMI PHARM IND KK) , 3 August 1993 & JP 00 594225 A
- PATENT ABSTRACTS OF JAPAN vol. 005, no. 132 (C-068), 22 August 1981 & JP 56 065816 A (GREEN CROSS CORP:THE), 3 June 1981

## Description

### FIELD OF THE INVENTION

The present invention relates to the preparation of pharmaceutical formulations with anti-ulcerative properties, and in particular, formulations that are reconstituted for intravenous administration.

### BACKGROUND OF THE INVENTION

Souda et al., U.S. Patent No. 5,045,552, describes compounds that inhibit an H⁺/K⁺-ATPase present in the stomach. These compounds are useful for treatment of peptic ulcers and other disorders associated with secretion of gastric acid, such as heartburn and gastroesophageal reflux. For example, one such compound has the following structure: 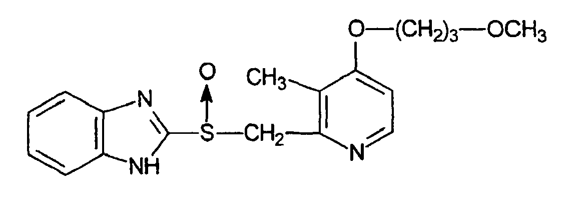 and includes pharmaceutically acceptable salts of the compound. This compound is referred to herein as Compound I.

JP 05 194225 A describes an aqueous solution of omeprazole, glycine and Na₂HPO₄ which is used to prepare tablets for oral dosage.

EP-A-0268956 describes benzimidazole compounds, including omeprazole. A formulation of these compounds suitable for intravenous injection is also described.

JP 56 065816 A describes the use of DL-form lysine to stabilize a powder pharmaceutical containing the active ingredient acetylsalicylate.

DE-A-3000743 describes the use of glycine for stabilizing a solid formulation of acetylsalicylate. The solid formulation may be subsequently dissolved and administered to patients.

EP-A-0082481 describes the use of glycine for stabilizing solid interferon formulations. The formulations may be reconstituted with water prior to administration.

It is desirable when preparing reconstituted solutions of such anti-ulcerative compounds that are suitable for intravenous administration, that the solubilized compounds exhibit physical and chemical stability for at least between 6 and 12 hours at room temperature. It has been found by the present inventors that anti-ulcerative compounds such as Compound 1 and the compounds described by general formula I below discolor when they are reconstituted, i.e., dissolved, in aqueous solutions, particularly in solutions suitable for intravenous administration, e.g., 5% dextrose or 0.9% saline. Such solutions quickly turn yellow to yellow-brown.

The compounds of the present invention have been determined to be more potent H⁺/K⁺-ATPase inhibitors than omeprazole sodium. However, in order to provide clinically useful pharmaceutical formulations of the compounds disclosed herein for intravenous administration, it is first necessary to provide formulations for lyophilization and intravenous administration that do not degrade physically, chemically and/or demonstrate a change in color.

### BRIEF DESCRIPTION OF THE DRAWINGS

Figure 1 is a graph showing the changes in absorption spectrum of compound 1 at a concentration of 4 mg/ml in 0.9 % saline at pH 10 as a function of time after dissolution, with storage at room temperature (25 °C) in the dark.
Figure 2 is a graph showing the changes in absorption spectrum of compound 1 at a concentration of 4 mg/ml in 0.9 % saline/50 mM glycine-NaOH buffer at pH 10 as a function of time after dissolution, with storage at room temperature (25 °C) in the dark.
Figure 3 is a graph showing the change in the absorption spectrum of compound 1, at a concentration of 4 mg/ml, in a solution which contain 5, 10, 25, and 50 mM glycine-NaOH buffer, indicating color change.
Figure 4 is a graph showing the change in the absorbance at 400, 450, 500, 550, 600, and 600 nm of compound 1, at a concentration of 2 mg/ml in 0.9% saline, at room temperature (25 °C) in the light, as a function of time.
Figure 5 is a graph showing the change in the absorbance at 400, 450, 500, 550, 600, and 600 nm of compound 1, at a concentration of 2 mg/ml in 0.9% saline, at room temperature (25 °C) in the dark, as a function of time.
Figure 6 is a graph showing the change in the absorbance at 400, 450, 500, 550, 600, and 600 nm of compound 1, at a concentration of 2 mg/ml in 0.9% saline, at 10 °C in the dark, as a function of time.
Figure 7 is a graph showing the change in the absorbance at 400, 450, 500, 550, 600, and 600 nm of compound 1, at a concentration of 2 mg/ml in 0.9% saline and 10 mM glycine-NaOH buffer, at room temperature (25 °C) in the light, as a function of time.
Figure 8 is a graph showing the change in the absorbance at 400, 450, 500, 550, 600, and 600 nm of compound 1, at a concentration of 2 mg/ml in 0.9% saline and 10 mM glycine-NaOH buffer, at room temperature (25 °C) in the dark, as a function of time.
Figure 9 is a graph showing the change in the absorbance at 400, 450, 500, 550, 600, and 600 nm of compound 1, at a concentration of 2 mg/ml in 0.9% saline and 10 mM gtycine-NaOH buffer, at 10 °C in the dark, as a function of time.

### DETAILED DESCRIPTION OF THE INVENTION

It has now been surprisingly and unexpectedly discovered that if lyophilized compounds of general formula I below are reconstituted in isotonic solutions suitable for intravenous administration, such as 5 % dextrose or 0.9% sodium chloride, that have been brought to a pH of between 9 and 12, preferably between pH 10 and 11, by a glycine-sodium hydroxide buffer, such formulations are chemically and physically stable, and do not significantly change color, for at least between 6 and 12 hours at room temperature. It was also discovered that the compounds dissolved in such isotonic solutions are stable to color change for from between 24 and 48 hours if kept at 5 °C. It has also been discovered that the use of glycine buffers with a pH of between 9 and 12, preferably between pH 10 and 11, is beneficial in preparing lyophilized samples of the compounds of the invention.

Thus, the present invention provides pharmaceutical formulations suitable for intravenous injection comprising an anti-ulcerative agent having the following general formula: 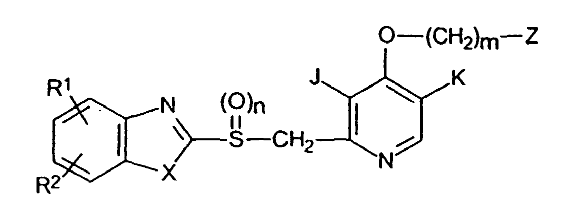 where R¹ and R² are, independently, hydrogen, C₁₋₆ alkyl, C₁₋₆ alkoxy, halogenated C₁₋₆ alkyl, C₁₋₆ alkoxycarbonyl or carboxyl group or a halogen atom;
Xis O, S or 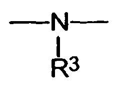 (in which R³ stands for a hydrogen atom or a C₁₋₆ alkyl, phenyl, benzyl or C₁₋₆ alkoxycarbonyl group); and
Z is selected from:
(1) a group of the formula:

   -O(CH₂)ₚ-O-R⁴

   where p is an integer of 1 to 3 and R⁴ is a hydrogen atom or a C₁-C₆ alkyl, aryl or aralkyl group;
(2) a group of the general formula:

   -O(CH₂)_{q}-R⁵

   where q is an integer of 1 to 3 and R⁵ is a halogen atom or an alkoxycarbonyl, aryl or heteroaryl group;
(3) a group of the general formula:

   -O(CH₂)ᵣ-O(CH₂)ₛ-R⁶

   where r and s each independently are an integer of 1 to 5 and R⁶ is a hydrogen atom or a C₁₋₆ alkyl group;
(4) a group of the formula: 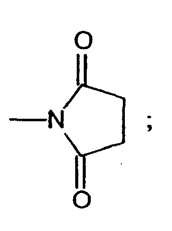
(5) a group of the formula: 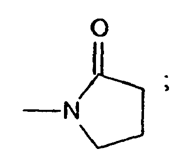
(6) a group of the formula: 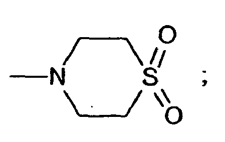
(7) a group of the general formula: 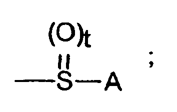 where t is an integer of 0 to 2 and A is a C₁₋₆ alkyl, alkoxycarbonylmethyl, pyridyl or furyl group, or a group of the general formula: 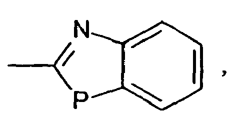 where P is selected from the group consisting of: -NH-, -O- or -S-; or a group of the general formula: 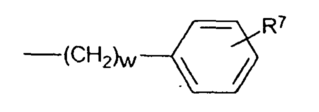 wherein R⁷ is hydrogen or C₁₋₆ alkyl and w is from 0 to 3;
(8) a group of the general formula: 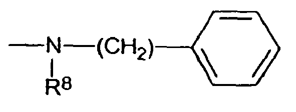 where R⁸ is an acetoxy or C₁₋₆ alkyl group; and
(9) a group of the general formula: -OR⁹
where R⁹ is a methyl group;
n is an integer of 0 to 2; m is an integer of 2 to 10, and
J and K are independently hydrogen or C₁₋₆ alkyl, with the proviso that when Z is a group falling under the above category (9), R⁹ is a methyl group and m stands for an integer of 3 to 10, and pharmaceutically acceptable salts thereof.

The pharmaceutical formulations also contain a glycine-sodium hydroxide buffer system, and an agent that imparts tonicity to the formulation (a "tonicity agent"). Such agents are well-known in the art, and include sodium chloride, dextrose, mannitol, glycerin, sucrose and lactose. Isotonic solutions posses the same osmotic pressure as blood plasma, and so can be intravenously infused into a subject without changing the osmotic pressure of the subject's blood plasma.

The definitions for R¹, R², X, n, J, K, Z and m are used consistently throughout the specification that follows and in the appended claims.

In the definition of the compounds of general formula (I), the lower alkyl group defined above with respect to R¹, R², R³, R⁴, R⁶, A, R⁷, R⁸, J, and K in compound (I) of the present invention may be straight-chain or branched alkyl groups having 1 to 6 carbon atoms. Examples include methyl, ethyl, n-propyl, n-butyl, isopropyl, isobutyl, 1-methylpropyl, tert-butyl, n-pentyl, 1-ethylpropyl, isoamyl, n-hexyl groups, among which methyl and ethyl groups are most preferred.

The lower alkoxy group and the lower alkoxy moiety of the lower alkoxycarbonyl group defined above with respect to R¹ and R² may be an alkoxy group derived form the above-defined and exemplified lower alkyl group. Methoxy and ethoxy groups are most preferred alkoxy groups.

The halogen atom defined above includes chlorine, bromine, iodine or fluorine. The aryl group defined above with respect to R⁴ and R⁵ may be phenyl, tolyl, xylyl, naphthyl which may be substituted with a C₁₋₆ alkoxy or hydroxyl group, a halogen atom.

The arylalkyl group defined above with respect to R⁴ is selected from benzyl and phenethyl groups.

The heteroaryl group defined above with respect to R⁵ is selected from pyridyl, furyl, and thienyl groups.

In the definition of Z in general formula (I), groups (1), (2), (3), (4), (5) and (9) are preferred; group (9) is the most preferred. R¹ and R² are preferably both hydrogen; another preferred configuration of R¹ and R² is when R¹ is C₁₋₆ alkyl, e.g., methyl, and R² is hydrogen. X is preferably -NR³ where R³ is hydrogen. A preferred value for n is 1. The preferred substituents for J and K are both hydrogen or, where J is C₁₋₆ alkyl, e.g. methyl, K is preferably hydrogen, and when J is hydrogen K is preferably C₁₋₆ alkyl, e.g. methyl. Thus, J or K are independently preferably hydrogen or methyl, most preferably J is methyl and K is hydrogen.

A first preferred class of compounds included in the pharmaceutical formulations of the present invention fall within the compounds of general formula (I) are represented by the following formula (A): 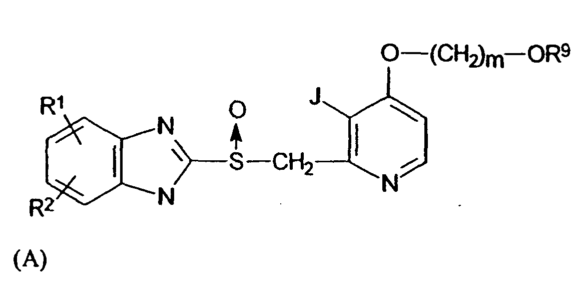 where R¹ and R² are independently selected from the group consisting of hydrogen, C₁₋₆ alkyl, C₁₋₆ alkoxy, halogenated C₁₋₆ alkyl, C₁₋₆ alkoxycarbonyl, a carboxyl group, and halogen; R⁹ is methyl, J is selected from the group consisting of hydrogen or C₁₋₆ alkyl; m is an integer from 2 to 10; and pharmaceutically acceptable salts thereof. In formula A, it is preferred that R¹ and R² are both hydrogen; also preferred is when R¹ is 5-C₁₋₆ alkoxy, 5-C₁₋₆ alkyl or 5-halogenated C₁₋₆ alkyl and R² is hydrogen. Preferred substituents at J are hydrogen or methyl; preferred values of m are from 3 to 10, the most preferred being 3.

In one group of preferred compounds of formula A, R¹ and R² are both hydrogen, J is methyl, m is 3 and R⁹ is methyl.

In a second group of preferred compounds falling within formula A, R¹ and R² are both hydrogen, J is hydrogen, m is 3 and R⁹ is methyl.

A second class of compounds falling within general formula (I) for inclusion in the pharmaceutical formulations of the present invention are represented by formula (B), as follows: 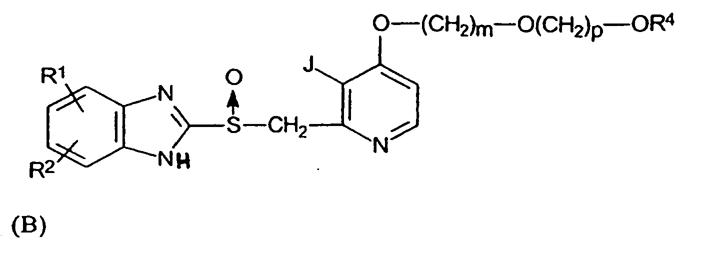 where R¹ and R² are independently selected from the group consisting of hydrogen, C₁₋₆ alkyl, C₁₋₆ alkoxy, halogenated C₁₋₆ alkyl, C₁₋₆ alkoxycarbonyl, a carboxyl group, and halogen; R⁴ is selected from the group consisting of hydrogen, C₁₋₆ alkyl, aryl, and aralkyl; J is selected from the group consisting of hydrogen or C₁₋₆ alkyl; m is an integer from 2 to 10; p is an integer from 1 to 3; and pharmaceutically acceptable salts thereof.

In compounds of formula (B), the preferred substituents for R¹ and R² are both hydrogen; also preferred are compounds where R¹ is 5-C₁₋₆ alkoxy, 5-C₁₋₆ alkyl or 5-halogenated C₁₋₆ alkyl and R² is hydrogen. Preferred values of m are 2 or 3; preferred values of p are 2 or 3; and the preferred substituents at R⁴ are methyl or benzyl. Most preferred are compounds of formula (B) where R¹ is 5-methyl, R² is hydrogen, J is methyl, m is 2, p is 2 and R⁴ is methyl.

Examples of the pharmaceutically acceptable salts include salts of inorganic acids, such as hydrochloride, hydrobromide, sulfate and phosphate; those with organic acids, such as acetate, maleate, tartrate, methanesulfonate, benzenesulfonate, and toluenesulfonate; and those with amino acids such as arginine, aspartic acid and glutamic acid.

Some of the compounds according to the present invention can form a salt with a metal such as Na, K, Ca or Mg. These metal salts are also included among the pharmaceutically acceptable salts of the present invention. For example, compounds represented by the general formula (I), wherein X is a group of 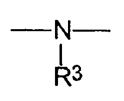 and R³ is a hydrogen atom, or compounds represented by the general formula (I), where Z is a group of category (7) and B is an NH group, can be present as a metal salt.

The compounds of the present invention also can take the form of hydrates, or stereoisomers. It will be appreciated by those of ordinary skill in the art that variations and obvious modifications can be made to the presently claimed invention, said variations and modifications being within the scope of the claimed invention.

Methods for the preparation of the compounds of the stabilized formulations of the invention are disclosed in Souda et al., U.S. Patent 5,045,552.

The present invention also provides methods for the stabilization of compounds of general formula I above, both in the course of preparing lyophilized samples for reconstitution, and in reconstituted formulations suitable for intravenous administration. Prior to the present invention, the utility of glycine as a color stabilizer for solutions of the compounds of the invention was not known in the art, either in the context of preparing solutions for lyophilization, or for preparing solutions for intravenous administration.

To prepare lyophilized samples for reconstitution, a desired quantity of a compound of the invention is dissolved in a sufficient amount of an aqueous solution (i.e., an amount of solution in which the compound will completely dissolve) which also comprises a glycine-sodium hydroxide buffer such that the pH of the solution is between 9 and 12, preferably between pH 10 and 11. The concentration of glycine in the solution is between 1 and 300 mM, preferably between 10 and 150 mM. The concentration of the compound of the invention in such solutions is generally from between 1 mg/ml and 50 mg/ml. The solution is then lyophilized in a sealable container, such as a vial, and the container is sealed such that exchange of air between the inside of the sealable container and the external environment of the container is not possible. The container will typically contain between 1 and 100 mg of the compound of the invention, preferably between 20 and 60 mg of the compound of the invention, and most preferably about 40 mg of the compound of the invention.

According to the present invention, reconstituted solutions for intravenous administration can be prepared by initially dissolving an amount of a desired lyophilized compound (plus any other solutes, such as glycine-NaOH buffer, which were lyophilized with the compound) in a sufficient amount of a sterile, aqueous solution to completely dissolve the lyophilized compound. Such initially dissolved solutions contain the original glycine-NaOH buffer system, substantially undiluted, and have a pH of from between 10 and 11.5. Under these conditions, as determined by the present inventors, the anti-ulcerative compounds of the invention are chemically and physically stable.

In order to deliver the compounds of the present invention intravenously, they may be dissolved in sterile solutions suitable for intravenous administration, such as normal saline (0.9% saline) or 5% dextrose. Such solutions typically have a pH of between 4 and 5, respectively. When the residual glycine-NaOH buffer system is diluted in the solution suitable for intravenous administration, for example a 50-fold dilution of 2 ml of a 20 mg/ml initial solution of anti-ulcerative compound, the pH of the resulting solution falls below the pH 9 to 12 range in which the anti-ulcerative compounds are most stable. Thus, according to the present invention, additional glycine-NaOH can be added to or included in the ultimate solution to be intravenously administered. The concentration of glycine-NaOH buffer in the final solution for intravenous administration should be between 1 mM and 300 mM, preferably between 10 mM and 150 mM, more preferably between 10 and 50 mM and most preferably between 10 mM and 25 mM. The pH of the resulting solution should be alkaline, preferably between pH 9 and 12, most preferably between pH 10 and 11.

The present invention is illustrated by the following examples.

### EXAMPLE 1: pH Studies

The chemical and physical stability of compound 1 at 8 mg/ml in a water for injection (WFI), adjusted with dilute 6M (6 N) NaOH to pH 9.5, 10, 11, and 11.5, was evaluated at room temperature, 5 °C, and -20 °C. Chemical stability was monitored by evaluating the residual potency and impurity levels over 48 hours by HPLC. Physical stability was evaluated by measuring the rate of color formation at 405 nm and by visual observations.

The order of chemical and physical stability is pH 11.5 > pH 11 > pH 10.5 > pH 10 > pH 9.5 at 5 °C and room temperature. That is, chemical and physical stability of compound 1 is highest at pH 11.5, and decreases with pH; this effect is found at room temperature and at reduced temperatures. Solutions at pH 9.5 began to assume a yellow color within 30 minutes; the color intensified rapidly. At room temperature, solutions at pH 10.5 were marginally stable at 24 hours with regard to chemical and physical stability; however, at cold temperatures (5°C), pH 10.5 was found to be adequate for 24 hours stability.

At pH 11 or greater and in cold temperatures, solutions of compound 1 appear to be adequately stable for the manufacture and handling in preparation for freeze drying. It was concluded that pH levels below 10.5 should be avoided.

### EXAMPLE 2: Preliminary Buffer Evaluation

It is desirable that the pH of solutions of compound 1 and other compounds of the invention in 5% dextrose or normal saline remain in a range near about pH 10 to provide for an acceptable use period in a clinical setting. Phosphate and glycine buffer systems were tested. Phosphate was found to be an effective buffer in the desired pH range, but, as indicated below, precipitated druing freeze-drying of samples containing it; glycine-NaOH was an effective buffer and had a stabilizing effect on color change and may affect turbidity when evaluated with compound 1.

Solutions of compound 1 in 50 mM phosphate buffer behaved similarly with regard to color formation as unbuffered compound 1 solutions (i.e., color formation was not inhibited). In 100 mM glycine/NaOH at pH values above 10, discoloration was substantially slower. Freeze-drying of compound 1 solutions in phosphate and glycine buffers yielded white, well-formed plugs. Reconstitution of the phosphate-containing plugs produced hazy solutions, i.e., precipitation. Based on this propensity to precipitate, phosphate was disqualified as a buffer for the compounds of the invention.

### EXAMPLE 3: Glycine Concentration and Temperature Studies

Compound 1 at 8 mg/ml in glycine at 0 mM, 100 mM, and 150 mM were evaluated at pH 10.5 to 11 at room temperature, 5 °C, and -20 °C. Chemical stability was monitored by measuring the residual potency and impurity levels over 48 hours. Physical stability was evaluated by measuring the rate of color formation at 405 nm and by visual observations. The results for color formation are shown in Tables 1, 2, and 3, below. A, B, and C contain 7.5 mg/ml glycine, equal to 100 mM glycine. D and E have 11.25 mg/ml glycine, equal to 150 mM glycine. F is the control without glycine. The pH of the solution is indicated in parentheses; the values in the tables are the absorbance at 405 nm.

**TABLE 1: COLOR INFORMATION ROOM TEMPERATURE (25 °C) SAMPLES (ABSORBANCE AT 405 nm)**

| | A(11.0) | B(10.76) | C(10.5) | D(11.0) | E(10.5) | F(10.5) |
|---|---|---|---|---|---|---|
| 0 hours | 0.009 | 0.010 | 0.011 | 0.008 | 0.011 | 0.012 |
| 6 hours | 0.034 | 0.048 | 0.066 | 0.032 | 0.056 | 0.188 |
| 12 hours | 0.053 | 0.076 | 0.107 | 0.047 | 0.089 | 0.349 |
| 24 hours | 0.101 | 0.145 | 0.200 | 0.091 | 0.162 | 0.838 |
| 48 hours | 0.163 | 0.245 | 0.333 | 0.152 | 0.269 | 2.396 |

**TABLE 2: REFRIGERATED SAMPLES (5 °C)**

| | A(11.0) | B(10.76) | C(10.5) | D(11.0) | E(10.5) | F(10.5) |
|---|---|---|---|---|---|---|
| 0 hours | 0.009 | 0.010 | 0.011 | 0.008 | 0.011 | 0.012 |
| 12 hours | 0.015 | 0.016 | 0.020 | 0.012 | 0.017 | 0.052 |
| 24 hours | 0.051 | 0.021 | 0.026 | 0.016 | 0.022 | 0.073 |
| 48 hours | 0.019 | 0.025 | 0.030 | 0.017 | 0.027 | 0.098 |

**TABLE 3: FROZEN SAMPLES (-20 °C)**

| | A(11.0) | B(10.76) | C(10.5) | D(11.0) | E(10.5) | F(10.5) |
|---|---|---|---|---|---|---|
| Initial | 0.009 | 0.010 | 0.011 | 0.008 | 0.011 | 0.012 |
| 24 hours | 0.011 | 0.012 | 0.0175 | 0.010 | 0.012 | 0.022 |
| 48 hours | 0.010 | 0.013 | 0.015 | 0.010 | 0.014 | 0.027 |

No substantial difference in chemical stability was noted between 0 mM, 100 mM, and 150 mM glycine formulations. Solutions with greater glycine concentrations discolored more slowly. Solutions devoid of glycine discolored very quickly regardless of temperature conditions. At 5 °C, pH 10.5 to 11 solutions can be held for 24 hours without measurable increases in impurity levels. At room temperature, there is a <0.5% increase in impurities for the pH 11 solution, but at pH 10.5, >1 % impurities were measured at 24 hours. Color formation at 5 °C is significantly retarded compared to room temperature. Cold temperatures, i.e., those at or near 5 °C, are also preferred for the manufacture of compound 1 and the other compounds of the invention.

### EXAMPLE 4: Reduced Glycine Concentration Experiments

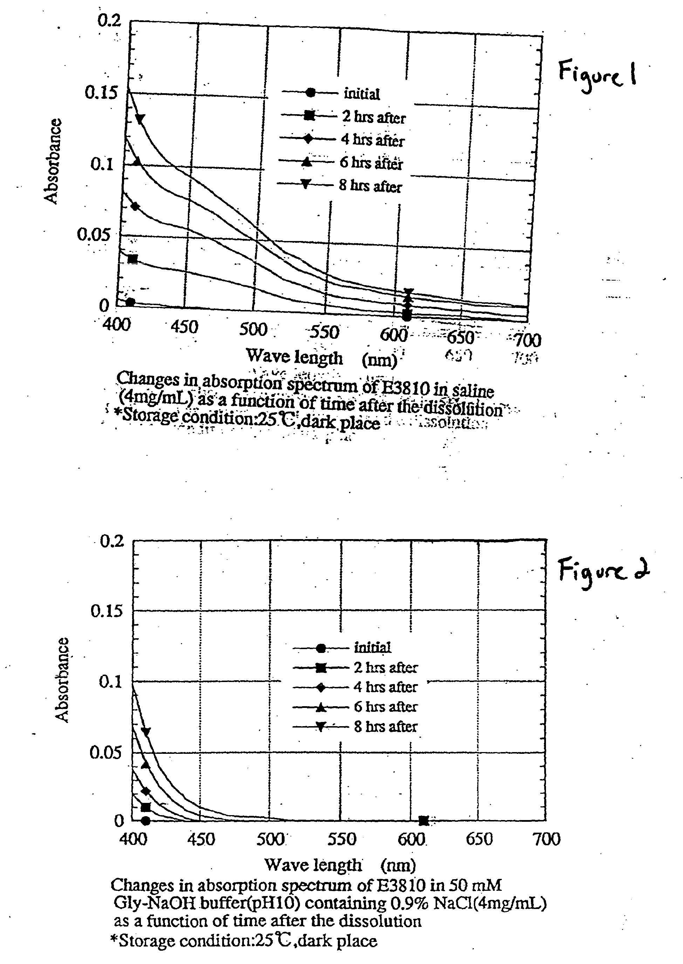

The color change in a 4 mg/ml solution of compound 1 in 0.9% saline at pH 10, with and without 50 mM glycine-NaOH buffer, was evaluated by measurement of absorption at 405 nm as a function of time. 200 mg of compound 1 was dissolved in 50 ml of 0.9% saline, and was stored at room temperature, i.e., 25 °C, in the dark. Absorption measurements were taken at the zero time point, and at 2, 4, 6, and 8 hours after dissolution. As can be seen from Figures 1 and 2, compound 1 discolored at a much greater rate in the glycine-free solution than in the solution that contained 50 mM glycine.

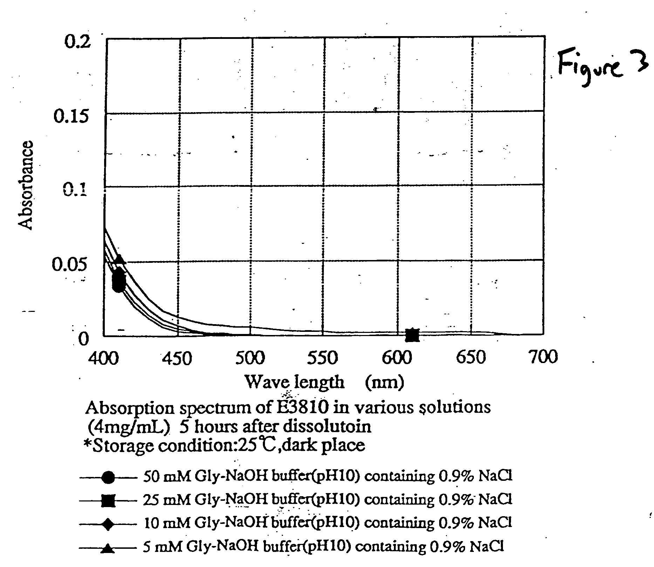

The glycine concentration-dependence of compound 1 discoloration was evaluated at 5 hours after dissolution. Compound 1 was dissolved at concentration of 4 mg/ml in 0.9% saline solution at pH 10 containing 5, 10, 25, and 50 mM glycine-NaOH buffer. As can be seen from Figure 3, at 5 hours post-dissolution, there was little difference in absorbance spectrum between the solutions, although there was a noticeably higher absorbance for the 5 mM glycine-NaOH containing solution.

### EXAMPLE 5: Effect of Storage Conditions

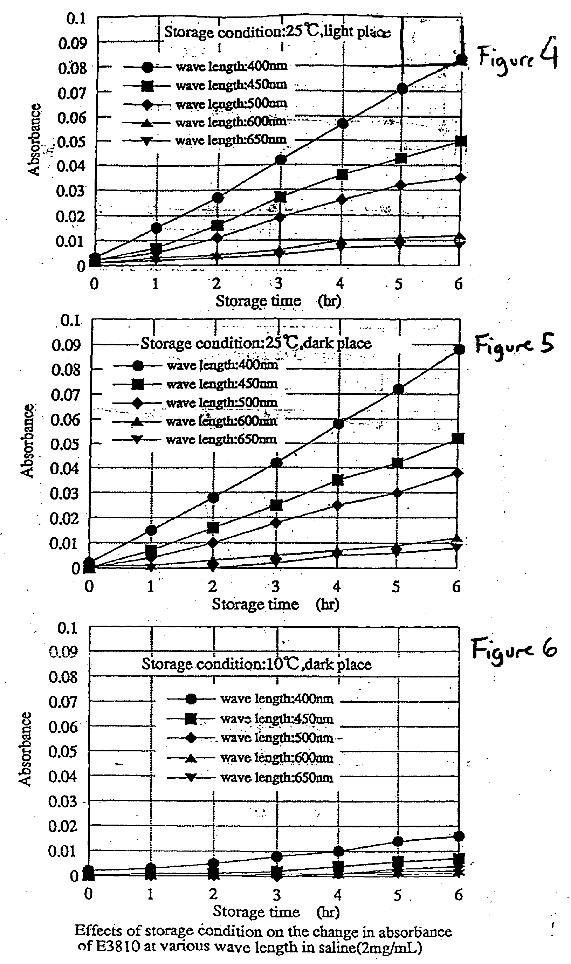

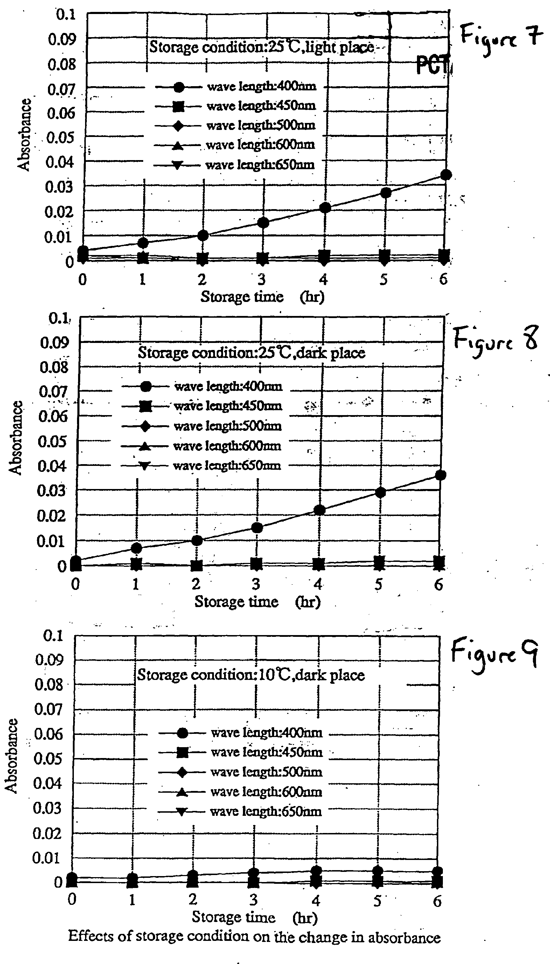

The effect of exposure to light and temperature was evaluated as a function of time for 0.9% saline solutions containing 2 mg/ml compound 1, with or without 10 mM glycine-NaOH buffer, was evaluated by monitoring absorbance at 400, 450 , 500, 600, and 650 nm. As can be seen from Figures 4 to 6, in solutions without glycine-NaOH buffer, increasing storage temperatures caused an increase in undesirable color development. The experiments also reveal that exposure to light has no detrimental effect on color development in solutions containing compound 1. These results are also found with solutions of compound 1 that do contain 10 mM glycine-NaOH buffer. However, as can be seen from Figures 7 to 9, the presence of glycine-NaOH buffer decreases absorption at all wavelengths, temperatures, and lighting conditions, i.e., glycine-NaOH buffer reduces color development in solutions of compound 1.

## Claims

1. An aqueous isotonic pharmaceutical formulation suitable for intravenous injection comprising:
an anti-ulcerative compound having the following formula: 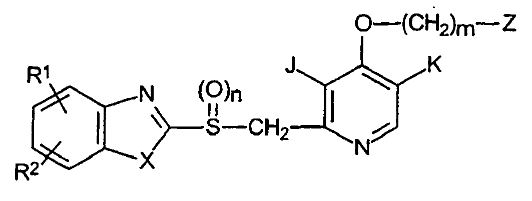
wherein R¹ and R² are independently selected from the group consisting of hydrogen, C₁₋₆ alkyl, C₁₋₆ alkoxy, halogenated C₁₋₆ alkyl, C₁₋₆ alkoxycarbonyl, a carboxyl group, and halogen;
X is a member selected from the group consisting of -O-, -S- or 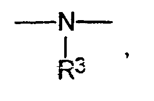 where R³ is a member selected from the group consisting of hydrogen, C₁₋₆ alkyl, phenyl, benzyl, and C₁₋₆ alkoxycarbonyl; and
Z is selected from the group consisting of:
(1) a group of the formula:
-O(CH₂)ₚ-O-R⁴
where p is an integer of 1 to 3 and R⁴ is a hydrogen atom or a C₁₋₆ alkyl, aryl or aralkyl group;
(2) a group of the general formula:
-O(CH₂)_{q}-R⁵
where q is an integer of 1 to 3 and R⁵ is a halogen atom or an alkoxycarbonyl, aryl or heteroaryl group;
(3) a group of the general formula:
-O(CH₂)ᵣ-O(CH₂)ₛ-R⁶
where r and s each independently are an integer of 1 to 5 and R⁶ is a hydrogen atom or a C₁₋₆ alkyl group;
(4) a group of the formula: 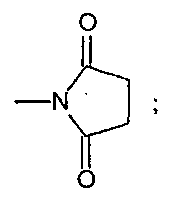
(5) a group of the formula: 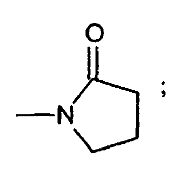
(6) a group of the formula: 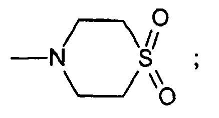
(7) a group of the general formula: 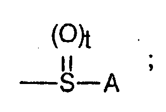 where t is an integer of 0 to 2 and A is a C₁₋₆ alkyl, alkoxycarbonylmethyl, pyridyl or furyl group, or a group of the general formula: 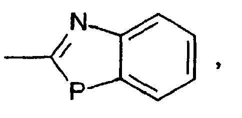 where P is selected from the group consisting of: -NH-, -O- or -S- or a group of the general formula: 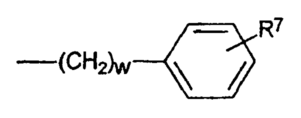 wherein R⁷ is hydrogen or C₁₋₆ alkyl and w is from 0 to 3;
(8) a group of the general formula: 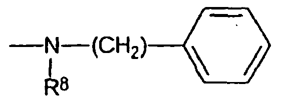 where R⁸ is an acetoxy or C₁₋₆ alkyl group; and
(9) a group of the general formula: -OR⁹
where R⁹ is a methyl group;
n is an integer of 0 to 2; m is an integer of 2 to 10, and
J and K are independently hydrogen or C₁₋₆ alkyl, with the proviso that when Z is a group falling under the above category (9), R⁹ is a methyl group and m stands for an integer of 3 to 10, and pharmaceutically acceptable salts thereof; and
a glycine buffer with a pH of between 9 and 12, in a pharmaceutically acceptable carrier;
wherein "aryl" means a phenyl, tolyl, xylyl or naphthyl group which may be substituted with a C₁₋₆ alkoxy, a hydroxy group or a halogen atom; "aralkyl" means a benzyl or phenethyl group; "heteroaryl" means a pyridyl, furyl or thienyl group.

2. An aqueous pharmaceutical formulation of claim 1 suitable for intravenous injection comprising:
an anti-ulcerative compound having the following formula: 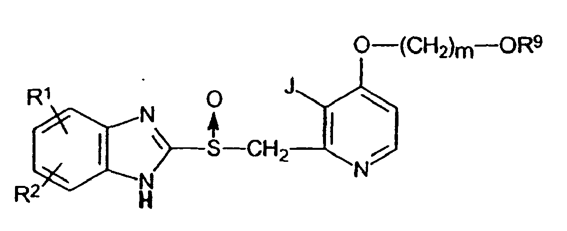
wherein R¹ and R² are independently selected from the group consisting of hydrogen, C₁₋₆ alkyl, C₁₋₆ alkoxy, halogenated C₁₋₆ alkyl, C₁₋₆ alkoxycarbonyl, a carboxyl group, and halogen;
wherein R⁹ is methyl;
wherein J is selected from the group consisting of hydrogen or C₁₋₆ alkyl;
wherein m is an integer from 3 to 10;
and pharmaceutically acceptable salts thereof;
glycine, sodium hydroxide; and
a tonicity agent.

3. An aqueous pharmaceutical formulation of claim 1 suitable for intravenous injection comprising:
an anti-ulcerative compound having the following formula: 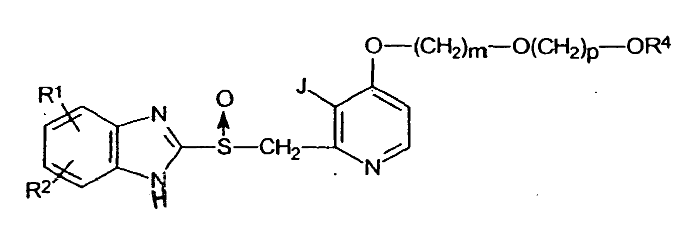
wherein R¹ and R² are independently selected from the group consisting of hydrogen, C₁₋₆ alkyl, C₁₋₆ alkoxy, halogenated C₁₋₆ alkyl, C₁₋₆ alkoxycarbonyl, a carboxyl group, and halogen;
wherein R⁴ is selected from the group consisting of hydrogen, C₁₋₆ alkyl, aryl, and aralkyl;
wherein J is selected from the group consisting of hydrogen or C₁₋₆ alkyl;
wherein m is an integer from 2 to 10;
wherein p is an integer from 1 to 3;
and pharmaceutically acceptable salts thereof;
glycine, sodium hydroxide; and
a tonicity agent;
wherein the terms "aryl" and "aralkyl" are as defined in claim 1.

4. The aqueous pharmaceutical formulation suitable for intravenous injection of claim 1 wherein said tonicity agent is selected from the group consisting of sodium chloride, glycerin, mannitol, sucrose, lactose, and dextrose.

5. The aqueous pharmaceutical formulation suitable for intravenous injection of claim 1 wherein said compound is

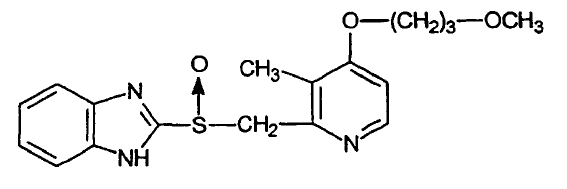

6. The aqueous pharmaceutical formulation suitable for intravenous injection of claim 2, 3 or 5 wherein said tonicity agent is selected from the group consisting of sodium chloride and dextrose.

7. The aqueous pharmaceutical formulation suitable for intravenous injection of claim 6 when dependent on claim 5 wherein said tonicity agent is sodium chloride and said sodium chloride is present in said formulation at a concentration of about 0.9% by weight.

8. The aqueous pharmaceutical formulation suitable for intravenous injection of claim 6 wherein said tonicity agent is dextrose and said dextrose is present in said formulation at a concentration of about 5% by weight.

9. The aqueous pharmaceutical formulation suitable for intravenous injection of claim 1 wherein said formulation has an alkaline pH, and wherein said glycine in said formulation is present at a concentration of between 1 mM and 300 mM.

10. The aqueous pharmaceutical formulation suitable for intravenous injection of claim 4 or 6 wherein said formulation has a pH of between 9 and 12, and wherein said glycine in said formulation is present at a concentration of between 10 mM and 300 mM.

11. A method for stabilizing anti-ulcerative formulations suitable for intravenous injection which comprises:
providing a compound as defined in claim 1 or 5, and pharmaceutically acceptable salts thereof;
providing a solution suitable for intravenous injection which has a pH of between 10 and 11 and which comprises glycine; and
admixing said compound and said solution until said compound is dissolved in said solution.

12. The method of claim 11 wherein said solution contains a solute selected from the group consisting of dextrose and sodium chloride.

13. The method of claim 11 wherein said glycine is present in said solution at a concentration of between 10 and 300 mM.

14. The method of claim 13 wherein said solution contains a solute selected from the group consisting of dextrose and sodium chloride, and wherein said solution is isotonic with blood plasma.

15. The formulation of claim 1, which comprises a tonicity agent.

16. The formulation of claim 1, which comprises sodium hydroxide.

17. The method of claim 11, wherein said alkaline pH is between 9 and 12.

## Patentansprüche

1. Wässrige isotonische pharmazeutische Formulierung, welche zur intravenösen Injektion geeignet ist, umfassend:
eine antiulcerative Verbindung mit der folgenden Formel: 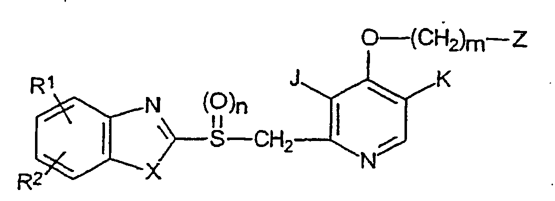
wobei R¹ und R² unabhängig aus der Gruppe, bestehend aus Wasserstoff, C₁₋₆ Alkyl, C₁₋₆ Alkoxy, halogeniertem C₁₋₆ Alkyl, C₁₋₆ Alkoxycarbonyl, einer Carboxylgruppe und Halogen, ausgewählt sind;
X ein Mitglied, ausgewählt aus der Gruppe, bestehend aus -O-, -S- oder 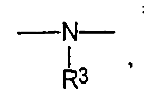 ist,
wobei R³ ein Mitglied, ausgewählt aus der Gruppe, bestehend aus Wasserstoff, C₁₋₆ Alkyl, Phenyl, Benzyl und C₁₋₆ Alkoxycarbonyl, ist, und
Z ausgewählt ist aus der Gruppe, bestehend aus:
(1) einer Gruppe der Formel:
-O(CH₂)ₚ-O-R⁴
wobei p eine ganze Zahl von 1 bis 3 und R⁴ ein Wasserstoffatom oder eine C₁₋₆ Alkyl-, Aryl- oder Aralkylgruppe ist,
(2) einer Gruppe der allgemeinen Formel:
-O(CH₂)_{q}-R⁵
wobei q eine ganze Zahl von 1 bis 3 und R⁵ ein Halogenatom oder eine Alkoxycarbonyl-, Aryl- oder Heteroarylgruppe ist;
(3) einer Gruppe der allgemeinen Formel:
-O(CH₂)ᵣ-O(CH₂)ₛ-R⁶
wobei r und s jeweils unabhängig eine ganze Zahl von 1 bis 5 sind und R⁶ ein Wasserstoffatom oder eine C₁₋₆ Alkylgruppe ist,
(4) einer Gruppe der Formel: 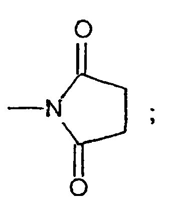
(5) einer Gruppe der Formel: 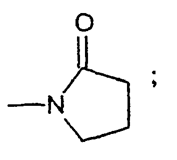
(6) einer Gruppe der Formel: 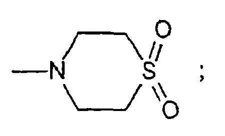
(7) einer Gruppe der allgemeinen Formel: 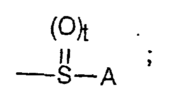 wobei t eine ganze Zahl von 0 bis 2 und A eine C₁₋₆ Alkyl-, Alkoxycarbonylmethyl-, Pyridyl- oder Furylgruppe ist, oder eine Gruppe der allgemeinen Formel: 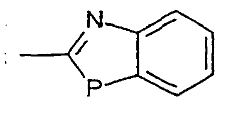 wobei P ausgewählt ist aus der Gruppe, bestehend aus: -NH-, -O- oder -S- oder einer Gruppe der allgemeinen Formel: 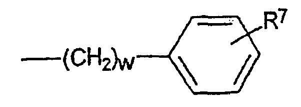 wobei R⁷ Wasserstoff oder C₁₋₆ Alkyl und w 0 bis 3 ist,
(8) einer Gruppe der allgemeinen Formel: 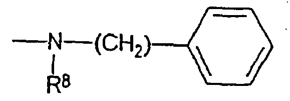 wobei R⁸ eine Acetoxy- oder C₁₋₆ Alkylgruppe ist, und
(9) einer Gruppe der allgemeinen Formel: -OR⁹
wobei R⁹ eine Methylgruppe ist,
n eine ganze Zahl von 0 bis 2 ist, m eine ganze Zahl von 2 bis 10 ist, und
J und K unabhängig Wasserstoff oder C₁₋₆ Alkyl sind, mit der Maßgabe, dass, wenn Z eine unter die vorstehende Kategorie (9) fallende Gruppe ist, R⁹ eine Methylgruppe ist und m für eine ganze Zahl von 3 bis 10 steht,
und pharmazeutisch verträgliche Salze davon; und
ein Glycin-Puffer mit einem pH zwischen 9 und 12, in einem pharmazeutisch verträglichen Träger,
wobei "Aryl" eine Phenyl-, Tolyl-, Xylyl- oder Naphthylgruppe bedeutet, welche mit einem C₁₋₆ Alkoxy, einer Hydroxygruppe oder einem Halogenatom substituiert sein kann;
"Aralkyl" eine Benzyl- oder Phenethylgruppe bedeutet;
"Heteroaryl" eine Pyridyl-, Furyl- oder Thienylgruppe bedeutet.

2. Wässrige pharmazeutische Formulierung nach Anspruch 1, welche zur intravenösen Injektion geeignet ist, umfassend:
eine antiulcerative Verbindung mit der folgenden Formel: 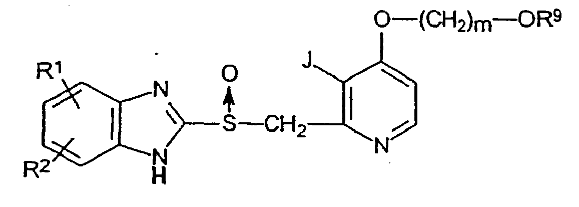
wobei R¹ und R² unabhängig aus der Gruppe, bestehend aus Wasserstoff, C₁₋₆ Alkyl, C₁₋₆ Alkoxy, halogeniertem C₁₋₆ Alkyl, C₁₋₆ Alkoxycarbonyl, einer Carboxylgruppe und Halogen, ausgewählt sind;
wobei R⁹ Methyl ist,
wobei J aus der Gruppe, bestehend aus Wasserstoff oder C₁₋₆ Alkyl, ausgewählt ist,
wobei m eine ganze Zahl von 3 bis 10 ist; und
pharmazeutisch verträgliche Salze davon,
Glycin, Natriumhydroxid; und
ein Tonizitätsmittel.

3. Wässrige pharmazeutische Formulierung nach Anspruch 1, welche zur intravenösen Injektion geeignet ist, umfassend:
eine antiulcerative Verbindung mit der folgenden Formel: 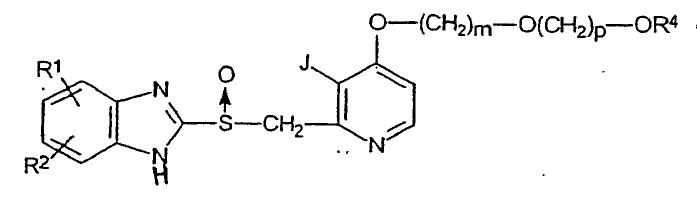
wobei R¹ und R² unabhängig aus der Gruppe, bestehend aus Wasserstoff, C₁₋₆ Alkyl, C₁₋₆ Alkoxy, halogeniertem C₁₋₆ Alkyl, C₁₋₆ Alkoxycarbonyl, einer Carboxylgruppe und Halogen, ausgewählt sind;
wobei R⁴ aus der Gruppe, bestehend aus Wasserstoff, C₁₋₆ Alkyl, Aryl und Aralkyl, ausgewählt ist,
wobei J aus der Gruppe, bestehend aus Wasserstoff oder C₁₋₆ Alkyl, ausgewählt ist,
wobei m eine ganze Zahl von 2 bis 10 ist,
wobei p eine ganze Zahl von 1 bis 3 ist; und
pharmazeutisch verträgliche Salze davon;
Glycin, Natriumhydroxid; und
ein Tonizitätsmittel;
wobei die Begriffe "Aryl" und "Aralkyl" wie in Anspruch 1 definiert sind.

4. Wässrige pharmazeutische Formulierung, geeignet zur intravenösen Injektion, nach Anspruch 1, wobei das Tonizitätsmittel aus der Gruppe, bestehend aus Natriumchlorid, Glycerin, Mannitol, Sucrose, Lactose und Dextrose, ausgewählt ist.

5. Wässrige pharmazeutische Formulierung, geeignet zur intravenösen Injektion, nach Anspruch 1, wobei die Verbindung 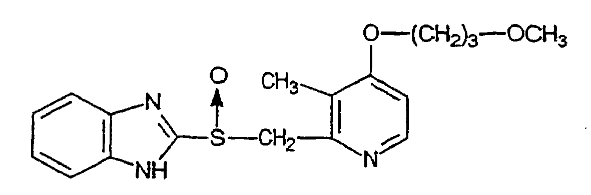 ist.

6. Wässrige pharmazeutische Formulierung, geeignet zur intravenösen Injektion, nach Anspruch 2, 3 oder 5, wobei das Tonizitätsmittel aus der Gruppe, bestehend aus Natriumchlorid und Dextrose, ausgewählt ist.

7. Wässrige pharmazeutische Formulierung, geeignet zur intravenösen Injektion, nach Anspruch 6, wenn abhängig von Anspruch 5, wobei das Tonizitätsmittel Natriumchlorid ist und das Natriumchlorid in der Formulierung in einer Konzentration von etwa 0,9 Gewichtsprozent vorliegt.

8. Wässrige pharmazeutische Formulierung, geeignet zur intravenösen Injektion, nach Anspruch 6, wobei das Tonizitätsmittel Dextrose ist und die Dextrose in der Formulierung in einer Konzentration von etwas 5 Gewichtsprozent vorliegt.

9. Wässrige pharmazeutische Formulierung, geeignet zur intravenösen Injektion, nach Anspruch 1, wobei die Formulierung einen alkalischen pH aufweist, und wobei das Glycin in der Formulierung in einer Konzentration zwischen 1 mM und 300 mM vorliegt.

10. Wässrige pharmazeutische Formulierung, geeignet zur intravenösen Injektion, nach Anspruch 4 oder 6, wobei die Formulierung einen pH zwischen 9 und 12 aufweist, und wobei das Glycin in der Formulierung in einer Konzentration zwischen 10 mM und 300 mM vorliegt.

11. Verfahren zur Stabilisierung antiulcerativer Formulierungen, geeignet zur intravenösen Injektion, umfassend:
das Bereitstellen einer Verbindung, wie in Anspruch 1 oder 5 definiert, und pharmazeutisch verträglicher Salze davon,
das Bereitstellen einer Lösung, die zur intravenösen Injektion geeignet ist, welche einen pH zwischen 10 und 11 aufweist und welche Glycin umfasst, und
das Vermischen der Verbindung und der Lösung bis die Verbindung in der Lösung gelöst ist.

12. Verfahren nach Anspruch 11, wobei die Lösung einen gelösten Stoff, ausgewählt aus der Gruppe, bestehend aus Dextrose und Natriumchlorid, enthält.

13. Verfahren nach Anspruch 11, wobei das Glycin in der Lösung in einer Konzentration zwischen 10 und 300 mM vorliegt.

14. Verfahren nach Anspruch 13, wobei die Lösung einen gelösten Stoff, ausgewählt aus der Gruppe, bestehend aus Dextrose und Natriumchlorid, enthält, und wobei die Lösung isotonisch mit Blutplasma ist.

15. Formulierung nach Anspruch 1, welche ein Tonizitätsmittel umfasst.

16. Formulierung nach Anspruch 1, welche Natriumhydroxid umfasst.

17. Verfahren nach Anspruch 11, wobei der alkalische pH zwischen 9 und 12 liegt.

## Revendications

1. Formulation pharmaceutique isotonique aqueuse convenant à une injection intraveineuse, comprenant :
un composé anti-ulcéreux ayant la formule suivante : 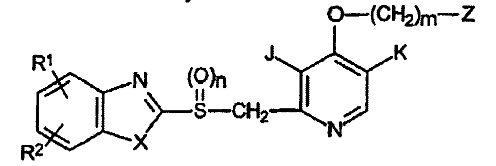
dans laquelle R¹ et R² sont chacun indépendamment de l'autre choisis dans l'ensemble consistant en l'atome d'hydrogène, les groupes alkyle en C₁₋₆, alcoxy en C₁₋₆, alkyle en C₁₋₆ halogéné, (alcoxy en C₁₋₆)carbonyle, le groupe carboxyle, et les halogènes ;
X est un membre choisi dans l'ensemble consistant en -O-, -S- ou 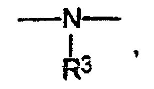 où R³ est un membre choisi dans l'ensemble consistant en l'atome d'hydrogène, les groupes alkyle en C₁₋₆, phényle, benzyle et (alcoxy en C₁₋₆)carbonyle ; et
Z est choisi dans l'ensemble consistant en :
(1) un groupe de formule : -O(CH₂)ₚ-O-R⁴, où p est un entier de 1 à 3, et R⁴ est un atome d'hydrogène ou un groupe alkyle en C₁₋₆, aryle ou aralkyle ;
(2) un groupe de formule générale : -O(CH₂)_{q}-R⁵, où q est un entier de 1 à 3, et R⁵ est un atome d'halogène ou un groupe alcoxycarbonyle, aryle ou hétéroaryle ;
(3) un groupe de formule générale : -O(CH₂)ᵣ-O(CH₂)ₛ-R⁶, où chaque indice r et s est indépendamment de l'autre un entier de 1 à 5, et R⁶ est un atome d'hydrogène ou un groupe alkyle en C₁₋₆ ;
(4) un groupe de formule : 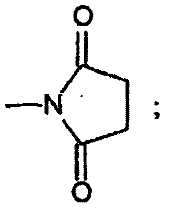
(5) un groupe de formule : 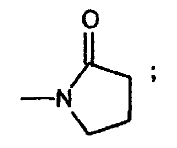
(6) un groupe de formule : 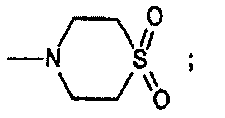
(7) un groupe de formule générale : 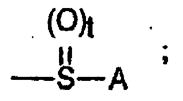 dans laquelle t est un entier de 0 à 2, et A est un groupe alkyle en C₁₋₆, alcoxycarbonylméthyle, pyridyle ou furyle, ou un groupe de formule générale : 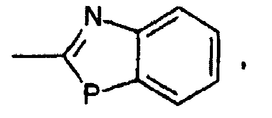 dans laquelle P est choisi dans l'ensemble consistant en -NH-, -O- ou -S-, ou un groupe de formule générale : 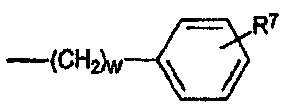 dans laquelle R⁷ est un atome d'hydrogène ou un groupe alkyle en C₁₋₆, et w vaut 0 à 3 ;
(8) un groupe de formule générale : 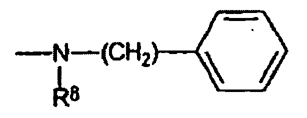 dans laquelle R⁸ est un groupe acétoxy ou alkyle en C₁₋₆ ; et
(9) un groupe de formule générale : -OR⁹, dans laquelle R⁹ est un groupe méthyle ; n est un entier de 0 à 2 ; m est un entier de 2 à 10, et J et K représentent chacun indépendamment de l'autre un atome d'hydrogène ou un groupe alkyle en C₁₋₆, à la condition que, quand Z est un groupe tombant dans la catégorie (9) ci-dessus, R⁹ soit un groupe méthyle et m représente un entier de 3 à 10, et ses sels acceptables d'un point de vue pharmaceutique ; et
un tampon glycine, ayant un pH compris entre 9 et 12, dans un excipient acceptable d'un point de vue pharmaceutique ;
où "aryle" désigne un groupe phényle, tolyle, xylyle ou naphtyle, qui peut être substitué par un groupe alcoxy en C₁₋₆, un groupe hydroxy ou un atome d'halogène ;
"aralkyle" désigne un groupe benzyle ou phénéthyle ;
"hétéroaryle" désigne un groupe pyridyle, furyle ou thiényle.

2. Formulation pharmaceutique aqueuse selon la revendication 1 convenant à une injection intraveineuse, comprenant :
un composé anti-ulcéreux ayant la formule suivante : 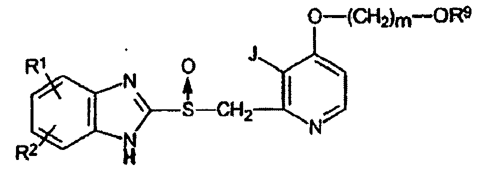
dans laquelle R¹ et R² sont chacun indépendamment de l'autre choisis dans l'ensemble consistant en l'atome d'hydrogène, les groupes alkyle en C₁₋₆, alcoxy en C₁₋₆, alkyle en C₁₋₆ halogéné, (alcoxy en C₁₋₆)carbonyle, un groupe carboxyle et les atomes d'halogène ;
où R⁹ est le groupe méthyle ;
où J est choisi dans l'ensemble consistant en l'atome d'hydrogène ou les groupes alkyle en C₁₋₆ ;
où m est un entier de 3 à 10 ;
et ses sels acceptables d'un point de vue pharmaceutique ;
de la glycine, de l'hydroxyde de sodium ; et un agent de tonicité.

3. Formulation pharmaceutique aqueuse selon la revendication 1, convenant à une injection intraveineuse, comprenant :
un composé anti-ulcéreux ayant la formule suivante : 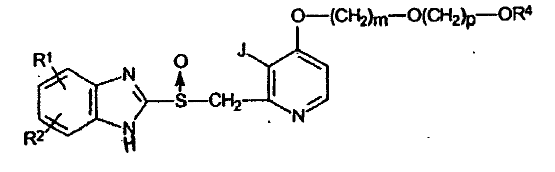
dans laquelle R¹ et R² sont chacun indépendamment de l'autre choisis dans l'ensemble consistant en l'atome d'hydrogène, les groupes alkyle en C₁₋₆, alcoxy en C₁₋₆, alkyle en C₁₋₆ halogéné, (alcoxy en C₁₋₆)carbonyle, un groupe carboxyle et les atomes d'halogène ;
où R⁴ est choisi dans l'ensemble consistant en l'atome d'hydrogène, les groupes alkyle en C₁₋₆, aryle et aralkyle ;
où J est choisi dans l'ensemble consistant en l'atome d'hydrogène ou des groupes alkyle en C₁₋₆ ;
où m est un entier de 2 à 10 ;
où p est un entier de 1 à 3 ;
et ses sels acceptables d'un point de vue pharmaceutique ;
de la glycine, de l'hydroxyde de sodium ; et un agent de tonicité ;
où les termes "aryle" et "aralkyle" sont tels que définis dans la revendication 1.

4. Formulation pharmaceutique aqueuse convenant à une injection intraveineuse selon la revendication 1, dans laquelle ledit agent de tonicité est choisi dans l'ensemble consistant en le chlorure de sodium, le glycérol, le mannitol, le saccharose, le lactose et le dextrose.

5. Formulation pharmaceutique aqueuse convenant à une injection intraveineuse selon la revendication 1, dans laquelle ledit composé est :

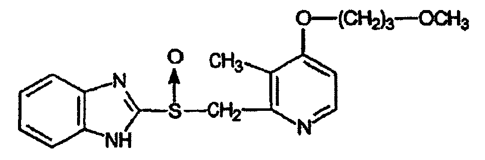

6. Formulation pharmaceutique aqueuse convenant à une injection intraveineuse selon la revendication 2, 3 ou 5, dans laquelle ledit agent de tonicité est choisi dans l'ensemble consistant en le chlorure de sodium et le dextrose.

7. Formulation pharmaceutique aqueuse convenant à une injection intraveineuse selon la revendication 6 quand elle dépend de la revendication 5, dans laquelle ledit agent de tonicité est le chlorure de sodium, et ledit chlorure de sodium est présent dans ladite formulation à une concentration d'environ 0,9 % en poids.

8. Formulation pharmaceutique aqueuse convenant à une injection intraveineuse selon la revendication 6, dans laquelle ledit agent de tonicité est le dextrose, et ledit dextrose est présent dans ladite formulation à une concentration d'environ 5 % en poids.

9. Formulation pharmaceutique aqueuse convenant à une injection intraveineuse selon la revendication 1, dans laquelle ladite formulation a un pH alcalin, et dans laquelle ladite glycine se trouvant dans ladite formulation est présente à une concentration de 1 à 300 mM.

10. Formulation pharmaceutique aqueuse convenant à une injection intraveineuse selon la revendication 4 ou 6, dans laquelle ladite formulation a un pH compris entre 9 et 12, et dans laquelle ladite glycine de ladite formulation est présente à une concentration comprise entre 10 et 300 mM.

11. Procédé pour stabiliser des formulations anti-ulcéreuses convenant à une injection intraveineuse, qui comprend :
la mise à disposition d'un composé tel que défini dans la revendication 1 ou 5, et de sels acceptables d'un point de vue pharmaceutique de ce composé ;
la mise à disposition d'une solution convenant à une injection intraveineuse, qui a un pH compris entre 10 et 11 et qui comprend de la glycine ; et
le mélange dudit composé et de ladite solution jusqu'à ce que ledit composé soit dissous dans ladite solution.

12. Procédé selon la revendication 11, dans lequel ladite solution contient un soluté choisi dans l'ensemble consistant en le dextrose et le chlorure de sodium.

13. Procédé selon la revendication 11, dans lequel ladite glycine est présente dans ladite solution à une concentration comprise entre 10 et 300 mM.

14. Procédé selon la revendication 13, dans lequel ladite solution contient un soluté choisi dans l'ensemble consistant en le dextrose et le chlorure de sodium, et dans lequel ladite solution est isotonique du plasma sanguin.

15. Formulation selon la revendication 1, qui comprend un agent de tonicité.

16. Formulation selon la revendication 1, qui comprend de l'hydroxyde de sodium.

17. Procédé selon la revendication 1, dans lequel ledit pH alcalin est compris entre 9 et 12.
